(19) Europäisches Patentamt
European Patent Office
Office européen des brevets

(11) **EP 3 097 201 B1**

(12) **FASCICULE DE BREVET EUROPEEN**

(45) Date de publication et mention
de la délivrance du brevet:
**28.11.2018 Bulletin 2018/48**

(51) Int Cl.:
***C12P 21/00*** *(2006.01)*   ***C12P 13/14*** *(2006.01)*

(21) Numéro de dépôt: **15704059.3**

(86) Numéro de dépôt international:
**PCT/FR2015/050123**

(22) Date de dépôt: **19.01.2015**

(87) Numéro de publication internationale:
**WO 2015/107312 (23.07.2015 Gazette 2015/29)**

(54) **PROCÉDÉ D'ENRICHISSEMENT EN PROTÉINES DE LA BIOMASSE DE MICROALGUES**

VERFAHREN ZUR ANREICHERUNG VON BIOMASSEPROTEINE

MICROALGAL BIOMASS PROTEIN ENRICHMENT METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.01.2014   FR 1450419**

(43) Date de publication de la demande:
**30.11.2016   Bulletin 2016/48**

(73) Titulaire: **Corbion Biotech, Inc.**
**South San Francisco, CA 94080 (US)**

(72) Inventeurs:
• **MACQUART, Gabriel**
  **F-62350 Mont Bernanchon (FR)**
• **DELAROCHE, Sylvain**
  **F-62219 Longuenesse (FR)**
• **LE RUYET, Marie**
  **59000 Lille (FR)**
• **SEGUEILHA, Laurent**
  **F-59520 Marquette Lez Lille (FR)**

(74) Mandataire: **Cabinet Becker et Associés**
**25, rue Louis le Grand**
**75002 Paris (FR)**

(56) Documents cités:
**EP-A1- 2 248 906     WO-A2-2014/154787**

• **SANSAWA H ET AL: "Production of intracellular phytochemicals in Chlorella under heterotrophic conditions", JOURNAL OF BIOSCIENCE AND BIOENGINEERING, ELSEVIER, AMSTERDAM, NL, vol. 98, no. 6, 1 janvier 2004 (2004-01-01), pages 437-444, XP004727088, ISSN: 1389-1723**

• **JIRI DOUCHA ET AL: "Production of high-densityculture grown in fermenters", JOURNAL OF APPLIED PHYCOLOGY, KLUWER ACADEMIC PUBLISHERS, DO, vol. 24, no. 1, 12 janvier 2011 (2011-01-12), pages 35-43, XP035001266, ISSN: 1573-5176, DOI: 10.1007/S10811-010-9643-2**

• **SHI X M ET AL: "High-yield production of lutein by the green microalga Chlorella protothecoides in heterotrophic fed-batch culture", BIOTECHNOLOGY PROGRESS, AMERICAN INSTITUTE OF CHEMICAL ENGINEERS, US, vol. 18, no. 4, 1 juillet 2002 (2002-07-01), pages 723-727, XP002689085, ISSN: 8756-7938, DOI: 10.1021/BP0101987 [extrait le 2002-07-17]**

• **JI YONGCHENG ET AL: "Differential effects of phosphorus limitation on cellular metals in Chlorella and Microcystis", LIMNOLOGY AND OCEANOGRAPHY, GRAFTON, WI, US, vol. 53, no. 5, 1 septembre 2008 (2008-09-01), pages 1790-1804, XP002729306, ISSN: 0024-3590**

• **CHUN-BO QU ET AL: "Phosphate assimilation by Chlorella and adjustment of phosphate concentration in basal medium for its cultivation", BIOTECHNOLOGY LETTERS, vol. 30, no. 10, 20 octobre 2008 (2008-10-20), pages 1735-1740, XP55179358, ISSN: 0141-5492, DOI: 10.1007/s10529-008-9758-6**

• **MALCOLM R. BROWN ET AL: "Biochemical composition of microalgae from the green algal classes Chlorophyceae and Prasinophyceae. 1. Amino acids, sugars and pigments", JOURNAL OF EXPERIMENTAL MARINE BIOLOGY AND ECOLOGY, vol. 161, no. 1, 1 octobre 1992 (1992-10-01), pages 91-113, XP55179365, ISSN: 0022-0981, DOI: 10.1016/0022-0981(92)90192-D**

**(Cont. page suivante)**

- RHEE G-Y: "EFFECTS OF NITROGEN PHOSPHORUS ATOMIC RATIOS AND NITRATE LIMITATION ON ALGAL GROWTH CELL COMPOSITION AND NITRATE UPTAKE", LIMNOLOGY AND OCEANOGRAPHY, vol. 23, no. 1, 1978, pages 10-25, XP002739689, ISSN: 0024-3590

- PLEISSNER DANIEL ET AL: "Effects of phosphorous, nitrogen, and carbon limitation on biomass composition in batch and continuous flow cultures of the heterotrophic dinoflagellate Crypthecodinium cohnii.", BIOTECHNOLOGY AND BIOENGINEERING AUG 2012, vol. 109, no. 8, août 2012 (2012-08), pages 2005-2016, XP002739690, ISSN: 1097-0290

**Description**

La présente invention se rapporte à un procédé d'enrichissement en protéines de la biomasse de microalgues, plus particulièrement du genre *Chlorella*, plus particulièrement encore de l'espèce *Chlorella sorokiniana* ou *Chlorella protothecoides*. La présente invention se rapporte également à un procédé d'enrichissement en protéines de la biomasse de certaines microalgues, plus particulièrement de *Chlorella protothecoides*, protéines dont la teneur en arginine et glutamine est remarquablement élevée.

Les algues, macro et micro, ont une richesse spécifique en grande partie inexplorée. Leur exploitation à des fins alimentaire, chimique ou bioénergétique est encore très marginale. Elles recèlent cependant des composants de grande valeur, en richesse comme en abondance.

Les microalgues sont en effet des sources de vitamines, lipides, protéines, sucres, pigments et antioxydants.

Les algues et microalgues intéressent ainsi le secteur industriel qui les utilise pour la fabrication de compléments alimentaires, d'aliments fonctionnels, de cosmétiques, de médicaments ou pour l'aquaculture.

Les microalgues sont avant tout des microorganismes photosynthétiques qui colonisent tous les biotopes exposés à la lumière.

A l'échelle industrielle, leur culture monoclonale est réalisée dans des photobioréacteurs (conditions autotrophiques : à la lumière avec du $CO_2$) ou pour certaines d'entre elles, également dans des fermenteurs (conditions hétérotrophiques : à l'obscurité en présence d'une source carbonée).

Quelques espèces de microalgues sont en effet capables de pousser en absence de lumière : *Chlorella, Nitzschia, Cyclotella, Tetraselmis, Crypthecodinium, Schizochytrium.*

Il est d'ailleurs estimé que la culture en conditions hétérotrophiques coûte 10 fois moins chère qu'en conditions phototrophiques car, pour l'Homme du métier, ces conditions hétérotrophiques autorisent :

- l'utilisation de fermenteurs identiques à ceux utilisés pour les bactéries et les levures et permettent le contrôle de tous les paramètres de culture.
- la production de biomasses en quantité bien plus élevée que ce qui est obtenu par culture basée sur la lumière.

L'exploitation rentable des microalgues nécessite généralement la maîtrise des conditions de fermentation permettant d'accumuler ses composants d'intérêt, tels que :

- les pigments (chlorophylle a, b et c, β-carotène, astaxanthine, lutéine, phycocyanine, xanthophylles, phycoérythrine...) dont la demande est croissante, tant pour leurs remarquables propriétés antioxydantes, que pour leur apport de couleurs naturelles dans l'alimentation,
- les lipides, ceci afin d'optimiser leur contenu en acides gras (jusqu'à 60 %, voir 80 % en poids de leur matière sèche) notamment pour :

  • les applications biocarburants, mais aussi
  • les applications en Alimentation humaine ou animale, lorsque les microalgues sélectionnées produisent des acides gras polyinsaturés ou PUFAs dits "essentiels" (i.e. apportés par l'alimentation car ne sont pas naturellement produits par l'homme ou l'animal), ou

- les protéines, ceci afin d'en optimiser les qualités nutritives ou par exemple de favoriser l'apport en acides aminés d'intérêt.

Dans le cadre de l'apport en acides aminés d'intérêt, il peut être en effet avantageux de disposer de sources de protéines riches en arginine et en glutamate

L'arginine est un acide aminé qui présente de nombreuses fonctions dans le règne animal.

L'arginine peut être dégradée et ainsi servir de source d'énergie, de carbone et d'azote à la cellule qui l'assimile.

Chez divers animaux, dont les mammifères, l'arginine est décomposée en ornithine et en urée. Cette dernière est une molécule azotée qui peut être éliminée (par excrétion dans les urines) de manière à réguler la quantité de composés azotés présente dans les cellules des organismes animaux.

L'arginine permet la synthèse du monoxyde d'azote (NO) par la NO synthétase, intervenant ainsi dans la vasodilatation des artères, ce qui réduit la rigidité des vaisseaux sanguins, augmente le flux sanguin et améliore ainsi le fonctionnement des vaisseaux sanguins.

Les compléments alimentaires qui contiennent l'arginine sont recommandés pour promouvoir la santé du coeur, la fonction vasculaire, pour prévenir « l'agrégation des plaquettes » (risque de formation de caillots sanguins) et pour abaisser la pression artérielle.

L'implication de l'arginine dans la cicatrisation des plaies est liée à son rôle dans la formation de la proline, un autre acide aminé important pour la synthèse de collagène.

L'arginine est enfin un composant fréquemment utilisé, notamment par les sportifs, dans les boissons énergisantes.

L'acide glutamique quant à lui n'est pas seulement l'une des briques élémentaires utilisées pour la synthèse des protéines, mais est aussi le neurotransmetteur excitateur le plus répandu dans le système nerveux central (encéphale + moelle épinière) et est un précurseur du GABA dans les neurones GABAergiques.

Sous le code de « E620 », le glutamate est utilisé comme exhausteur de goût des aliments. Il est additionné aux préparations alimentaires pour renforcer leur goût.

Outre le glutamate, le Codex Alimentarius a reconnu aussi comme exhausteurs de goût ses sels de sodium (E621), de potassium (E622), calcium (E623), ammonium (E624) et magnésium (E625).

Le glutamate (ou ses sels) est souvent présent dans les plats tout prêts (soupes, sauces, chips, plats cuisinés). Il est aussi couramment utilisé en cuisine asiatique.

Il est actuellement fréquemment utilisé en combinaison avec des arômes dans les apéritifs (goût bacon, goût fromage). Cela permet de rehausser le goût de bacon, du fromage, etc. Il est rare de trouver un apéritif qui n'en contienne pas.

On en trouve aussi dans certaines capsules de médicaments mais pas pour ses fonctions gustatives.

Enfin, il est le composant majoritaire des auxiliaires de cuisine (bouillons cubes, fonds de sauces, sauces, etc.).

Pour parvenir à exploiter les richesses métaboliques des microalgues, de premiers procédés de fermentation permettant d'obtenir de hautes densités cellulaires (acronyme anglais : HCD pour *High-Cell-Density*) ont été ainsi beaucoup travaillés, de manière à obtenir des rendements et productivités maximales en protéines ou en lipides.

L'objectif de ces cultures HCD était l'obtention de la concentration la plus élevée possible du produit souhaité dans le laps de temps le plus court.

Ce précepte se vérifie par exemple pour la biosynthèse d'astaxanthine par *Chlorella zofingiensis,* où la croissance de la microalgue s'est avérée corrélée directement avec la production de ce composé (Wang and Peng, 2008, World J Microbiol. Biotechnol., 24(9), 1915-1922).

Cependant, le fait de maintenir la croissance à son taux maximal ($\mu$, en $h^{-1}$) n'est pas toujours corrélé avec la production élevée du produit souhaité.

Il est en effet vite apparu aux spécialistes du domaine qu'il faut par exemple soumettre les microalgues à un stress nutritionnel qui limite leur croissance lorsqu'on souhaite leur faire produire d'importantes réserves lipidiques.

Il est donc procédé désormais au découplage croissance / production dans les procédés fermentaires.

Par exemple, pour favoriser l'accumulation d'acides gras polyinsaturés (ici l'acide docosahexaénoïque ou DHA), la demande de brevet WO 01/54510 recommande de dissocier la croissance cellulaire et la production d'acides gras polyinsaturés.

Il y est plus particulièrement revendiqué un procédé pour la production de lipides microbiens, comprenant les étapes consistant à :

(a) effectuer une fermentation d'un milieu comprenant des microorganismes, une source de carbone et une source nutritive limitante et assurant des conditions suffisantes pour maintenir un taux d'oxygène dissous d'au moins environ 4 % de la saturation dans ledit milieu de fermentation pour augmenter la biomasse ;

(b) puis fournir des conditions suffisantes pour maintenir un taux d'oxygène dissous approximativement égal ou inférieur à 1 % de la saturation dans ledit milieu de fermentation et fournir des conditions suffisantes pour permettre auxdits microorganismes de produire lesdits lipides ;

(c) et recueillir lesdits lipides microbiens, dans lequel au moins environ 15 % desdits lipides microbiens sont constitués de lipides polyinsaturés ;

et dans lequel une densité de biomasse d'au moins environ 100 g/l est obtenue au cours de la fermentation.

Chez la microalgue *Schizochytrium sp* souche ATCC 20888, il est ainsi plus particulièrement procédé à une première phase de croissance en présence d'une source carbonée et d'une source azotée mais sans limitation en oxygène, de manière à favoriser l'obtention d'une haute densité cellulaire puis, dans une deuxième phase, arrêter la fourniture d'azote et ralentir progressivement l'apport en oxygène (gestion de la pression en oxygène dissous ou $pO_2$ de 10 %, à 4 %, puis 0,5 %), afin de stresser la microalgue, ralentir sa croissance et enclencher la production des acides gras d'intérêt.

Chez la microalgue *Crypthecodinium cohnii*, la teneur la plus élevée en acide docosahexaénoïque (DHA, acide gras polyinsaturé) est obtenue à faible concentration en glucose (de l'ordre de 5 g/l), et ainsi à faible taux de croissance (Jiang and Chen, 2000, Process Biochem., 35(10), 1205-1209).

Ces résultats illustrent bien le fait que les cinétiques de formation des produits peuvent aussi bien être associées de façon positive que de façon négative avec la croissance des microalgues, voire même avec une combinaison des deux.

De ce fait, dans les cas où la formation des produits n'est pas corrélée avec une croissance cellulaire élevée, il est judicieux de maîtriser le taux de croissance cellulaire.

En général, l'homme du métier choisit de contrôler la croissance des microalgues par la maîtrise des conditions de fermentation (Tp, pH...), ou par l'alimentation régulée en composants nutritionnels du milieu de fermentation (conditions semi continues dites « fed-batch »).

S'il choisit de contrôler la croissance des microalgues en hétérotrophie par l'apport en sources carbonées, l'homme du métier choisit généralement d'adapter la source carbonée (glucose pur, acétate, éthanol...) à la microalgue (*C. cohnii*, *Euglena gracilis*...) en fonction du métabolite produit (par exemple un acide gras polyinsaturé de type DHA). La température peut-être également un paramètre clef :

- il a par exemple était rapporté que la synthèse d'acides gras polyinsaturés chez certaines espèces de microalgues, tel que l'EPA par *Chlorella minutissima*, est favorisée à une plus basse température que celle requise pour la croissance optimale de ladite microalgue;
- au contraire le rendement en lutéine est plus élevé chez *Chlorella protothecoides* cultivée en hétérotrophie, lorsque l'on augmente la température de production de 24 à 35°C.

*Chlorella protothecoides* est justement reconnue comme une des meilleures microalgues productrices d'huile.

En conditions hétérotrophiques, elle transforme rapidement les hydrates de carbone en triglycérides (plus de 50 % de sa matière sèche).

Pour optimiser cette production en triglycérides, l'homme du métier est amené à optimiser le flux carboné vers la production d'huile, en agissant sur l'environnement nutritionnel du milieu de fermentation.

Il est ainsi connu que l'accumulation en huile se produit lors d'un apport carboné suffisant, mais dans des conditions de carence en azote.

Le rapport C/N est donc ici déterminant, et il est admis que les meilleurs résultats sont obtenus en agissant directement sur la teneur en azote, la teneur en glucose n'étant pas limitante.

De manière non surprenante, cette carence en azote affecte la croissance cellulaire, ce qui résulte en un taux de croissance de 30 % plus faible que le taux de croissance normal de la microalgue (Xiong et al., Plant Physiology, 2010, 154, pp1001-1011).

Pour expliquer ce résultat, Xiong et al, dans l'article cité supra, démontrent en effet que si l'on divise la biomasse de *Chlorella* en ses 5 composants principaux, i.e. hydrates de carbone, lipides, protéines, ADN et ARN (représentant 85 % de sa matière sèche), si le rapport C/N n'a aucun impact sur la teneur en ADN, ARN et hydrates de carbone, il devient prééminent pour le contenu en protéines et en lipides.

C'est ainsi que les cellules de Chlorelles cultivées avec un rapport C/N faible contiennent 25,8 % de protéines et 25,23 % en lipides, tandis qu'un rapport C/N élevé permet la synthèse de 53,8 % de lipides et 10,5 % de protéines.

Pour optimiser sa production en huile, il est donc primordial pour l'homme du métier de contrôler le flux carboné en le déviant vers la production d'huile, au détriment de la production des protéines ; le flux carboné est redistribué et s'accumule en substances de réserve lipidiques quand les microalgues sont placées en milieu carencé en azote.

Fort de cet enseignement, pour la production de biomasses riches en protéines, l'homme du métier est donc amené à prendre l'opposé de ce contrôle métabolique, i.e. travailler les conditions de fermentation en favorisant plutôt un rapport C/N faible, et ainsi :

- réaliser un apport important en source d'azote au milieu de fermentation, tout en maintenant constant la charge en source carbonée qui sera convertie en protéines et
- stimuler la croissance de la microalgue.

Il s'agit de modifier le flux carboné vers la production de protéines (et donc de biomasses), au détriment de la production des lipides de réserve.

SANSAWA et al. (2004, Journal of Bioscience and Bioengineering, 98, 437-444) décrit un procédé de culture en hétérotrophie d'une microalgue, *Chlorella regularis*, comportant une étape visant à limiter la croissance de ladite microalgue par une carence totale d'alimentation en glucose du milieu de fermentation, permettant ainsi d'atteindre une teneur en protéines de la biomasse de 63 % en poids.

WO2014154787, publiée postérieurement au dépôt de la présente demande, décrit un procédé d'enrichissement en protéines d'une microalgue cultivée en hétérotrophie, microalgue du genre *Chlorella*, procédé de culture hétérotrophique qui comporte une étape visant à limiter la croissance de ladite microalgue par une carence du milieu de fermentation en glucose.

Dans le cadre de l'invention, la société Demanderesse a au contraire choisi d'explorer une voie originale en proposant une solution alternative à celle classiquement envisagée par l'homme du métier.

L'invention est relative ainsi sur un procédé d'enrichissement en protéines d'une microalgue cultivée en hétérotrophie, microalgue du genre *Chlorella*, plus particulièrement encore *Chlorella sorokiniana* ou *Chlorella protothecoides*, procédé de culture hétérotrophique qui comporte une étape visant à limiter la croissance de ladite microalgue par une carence du milieu de fermentation en glucose.

Cette étape est une étape de culture hétérotrophique où le glucose est apporté en quantité insuffisante dans le milieu pour permettre la croissance de la microalgue.

Au sens de l'invention, « l'enrichissement » s'entend d'une augmentation de la teneur en protéines de la biomasse pour atteindre une teneur en protéines de la biomasse de plus de 50 %, 60%, 65% ou 70% en poids.

L'invention est plus précisément relative à un procédé de culture hétérotrophique desdites microalgues comportant une étape visant à limiter la croissance de ladite microalgue par une carence du milieu de fermentation en glucose. La température de fermentation est modifiée de façon à ralentir la croissance de la microalgue. La température de fermentation peut notamment être augmentée de 1, 2, 3, 4 ou 5 degrés par rapport à la température optimale de fermentation qui est habituellement d'environ 28°C. De préférence, la température de fermentation est augmentée d'environ 3°C, par exemple en passant de 28°C à 31°C.

Tel qu'utilisé ici, le terme « environ » se réfère à une valeur +/- 20%, 10%, 5% ou 2%. Ainsi la présente invention est relative à un procédé d'enrichissement en protéines d'une microalgue cultivée en hétérotrophie, microalgue du genre *Chlorella*, plus particulièrement encore *Chlorella sorokiniana* ou *Chlorella protothecoides*, le procédé comprenant la culture hétérotrophique qui comporte une étape visant à limiter la croissance de ladite microalgue par une carence du milieu de fermentation en glucose, permettant ainsi d'atteindre une teneur en protéines de la biomasse de plus de 50, 60 ou 70 % en poids.

Par « carence du milieu de fermentation en glucose » on entend une culture dans laquelle le glucose est apportée à la microalgue en quantité insuffisante pour permettre sa croissance. Cette carence se traduit par un taux de croissance de la microalgue inférieur à celui de ladite microalgue en absence de limitation nutritionnelle.

Cela se traduit par une absence de glucose résiduel dans le milieu de culture, la microalgue consommant ce facteur nutritif au fur et à mesure de son apport.

Au sens de l'invention, le critère essentiel est donc bien la limitation de la croissance cellulaire induite par un stress, stress cellulaire provoquée par la carence en glucose du milieu de fermentation.

Cette stratégie va donc bien à l'encontre du préjugé technique selon lequel pour augmenter la teneur en protéines de la biomasse, il faut immanquablement augmenter cette biomasse et donc la croissance cellulaire.

Comme il sera exemplifié ci-après, on peut choisir avantageusement de limiter la croissance de *Chlorella sorokiniana* par une carence en glucose du milieu de fermentation.

De manière optionnelle, la limitation de la croissance de ladite microalgue peut être obtenue par l'ajout dans le milieu de culture de substances inhibitrices de la croissance cellulaire, comme les sulfates.

Le ralentissement de la croissance de ladite microalgue est également suscité en modifiant la température de fermentation, par exemple en l'augmentant de 1 à 5°C environ, de préférence d'environ 3°C, par rapport à la température optimale de fermentation. Cette température optimale de fermentation est habituellement d'environ 28°C.

Par ailleurs, sans être liée par une quelconque théorie, la société Demanderesse a trouvé que le flux de glucose est normalement utilisé chez les microalgues du genre *Chlorella sorokiniana* selon un ordre de priorité bien précis :

1. le métabolisme basal,
2. la croissance, i.e. formation d'une biomasse riche en protéines,
3. les substances de réserve (lipides et hydrates de carbone tel l'amidon).

Ce principe permet d'expliquer les variations naturelles de teneur en protéines au cours de la croissance de la microalgue, malgré l'apport constant en azote.

La société Demanderesse a ainsi trouvé que, pour enrichir la biomasse de microalgues en protéines, il faut limiter la croissance de la microalgue et contrôler sa consommation en source nutritive autre que l'azote, par exemple en glucose de manière à :

- dédier la consommation en glucose toute entière aux voies de production de protéines,
- éviter l'accumulation de substance de réserve comme les lipides.

En effet, éviter une carence en azote permet d'empêcher la déviation des flux métaboliques vers la production de lipides.

De manière optionnelle, il peut être avantageux d'aller jusqu'à bloquer complètement toute synthèse de matériel de réserve, en agissant par l'intermédiaire d'inhibiteurs spécifiques.

Il est connu en effet un certain nombre d'inhibiteurs des voies de synthèse des lipides voire même de l'amidon (hydrate de carbone de réserve par excellence des microalgues vertes) :

- pour les lipides, la cérulénine est décrite comme inhibiteur de la synthèse des acides gras, ou la lipstatine, substance naturelle produite par *Streptomyces toxytricini*, comme inhibiteur des lipases...

- pour l'amidon, les iminosucres (obtenu par simple substitution de l'atome d'oxygène endocyclique des sucres par un atome d'azote) sont historiquement connus en tant qu'inhibiteurs puissants des glycosidases, des glycosyltransférases, des glycogènes phosphorylases, ou de l'UDP-Galp mutase.

Ainsi, le procédé comprend la fermentation d'une biomasse de microalgues en conditions hétérotrophiques avec une première étape de croissance de la biomasse et avec une deuxième étape de carence du milieu de fermentation en glucose.

Cette deuxième étape permet d'enrichir la biomasse en protéine. En particulier, elle permet d'atteindre une teneur en protéines de la biomasse de plus de 50, 60, 65 ou 70 % en poids (en poids de matière sèche).

Dans un premier mode préféré de réalisation conforme à l'invention, le procédé de culture hétérotrophique desdites microalgues, notamment *Chlorella sorokiniana*, comprend:

∘ une première étape de croissance des microalgues, où la quantité de glucose dans le milieu (en mode discontinu) ou le débit d'apport en glucose (en mode continu ou semi-continu) est ajusté à leur capacité de consommation, de façon à obtenir rapidement une biomasse importante,
∘ une deuxième étape de production de protéines par les microalgues, où le débit d'apport en glucose est fixé à une valeur nettement en dessous de leur capacité de consommation du glucose, de façon à empêcher l'accumulation supplémentaire de substances de réserve, voire favoriser leur consommation.

Comme il sera exemplifié ci-après, la première étape de croissance des microalgues peut être réalisée en mode discontinu ou "batch", dans lequel le glucose apporté initialement est entièrement consommé par la microalgue, conduisant à la production d'une biomasse de base.

La deuxième étape de production de protéines est conduite dans des conditions où on réalise :

a) soit un apport de milieu complet en mode semi-continu, dit « fed-batch », après la consommation du glucose apporté initialement ; les autres paramètres de conduite de la fermentation sont inchangés. Dans ce cas, le glucose est apporté en continu et la vitesse d'apport est alors inférieure à la vitesse de consommation que la souche pourrait réaliser de sorte que la teneur résiduelle en glucose dans le milieu est maintenue nulle. La croissance de la souche est alors limitée par la disponibilité en glucose (condition glucose-limitant).

b) un fonctionnement en continu de type chemostat, dans lequel le taux de croissance de la souche ($\mu$) est maintenu à sa valeur minimale, la croissance de la souche étant limitée par l'apport en glucose. Ce mode de fonctionnement permet d'obtenir une biomasse à forte teneur en protéines grâce à la limitation en glucose et au faible taux de croissance imposé tout en assurant une très bonne productivité.

L'invention sera mieux comprise à l'aide des exemples qui suivent, lesquels se veulent illustratifs et non limitatifs.

## Exemples

### Exemple 1 : Production de *Chlorella sorokiniana* en fermentation de type « batch séquentiel » sans limitation de l'apport en milieu nutritif

La souche utilisée est une *Chlorella sorokiniana* (souche UTEX 1663 - *The Culture Collection of Algae at the University of Texas at Austin* - USA).

*Préculture :*

- 600 mL de milieu dans un Erlenmeyer de 2 L ;
- Composition du milieu (tableau 1 ci-dessous)

Tableau 1.

| Macro éléments (g/L) | Glucose | 20 |
|---|---|---|
| | $K_2HPO_4.3H2O$ | 0,7 |
| | $MgSO_4.7H2O$ | 0,34 |
| | Acide citrique | 1,0 |
| | Urée | 1,08 |
| | $Na_2SO_4$ | 0,2 |
| | $Na_2CO_3$ | 0,1 |
| | clerol FBA 3107 (antimousse) | 0,5 |
| Micro éléments (mg/L) | $Na_2EDTA$ | 10 |
| | $CaCl_2.2H2O$ | 80 |
| | $FeSO_4.7H2O$ | 40 |
| | $MnSO_4.4H2O$ | 0,41 |
| | $CoSO_4.7H2O$ | 0,24 |
| | $CuSO_4.5H2O$ | 0,24 |
| | $ZnSO_4.7H2O$ | 0,5 |
| | $H_3BO_3$ | 0,11 |
| | $(NH_4)_6Mo_7O_{27}.4H2O$ | 0,04 |

Le pH est ajusté à 7 avant stérilisation par ajout de NaOH 8N. L'incubation se déroule dans les conditions suivantes :

- durée : 72 h ;
- température : 28°C ;
- agitation : 110 rpm (Incubateur Infors Multitron).

La préculture est ensuite transférée dans un fermenteur de 30L de type Sartorius.

*Culture pour production de biomasse :*

Le milieu est identique à celui de la préculture, mais l'urée est remplacée par du $NH_4Cl$.

Tableau 2.

| Macro éléments (g/L) | Glucose | 20 |
|---|---|---|
| | $K_2HPO_4.3H2O$ | 0,7 |
| | $MgSO_4.7H2O$ | 0,34 |
| | Ac Citrique | 1,0 |
| | $NH_4Cl$ | 1,88 |
| | $Na_2SO_4$ | 0,2 |

| | | |
|---|---|---|
| | clerol FBA 3107 (antimousse) | 0,5 |
| Micro éléments (mg/L) | Na$_2$EDTA | 10 |
| | CaCl$_2$ | 80 |
| | FeSO$_4$.7H2O | 40 |
| | MnSO$_4$.4H2O | 0,41 |
| | CoSO$_4$.7H2O | 0,24 |
| | CuSO$_4$.5H2O | 0,24 |
| | ZnSO$_4$.7H2O | 0,5 |
| | H$_3$BO$_3$ | 0,11 |
| | (NH$_4$)$_6$Mo$_7$O$_{27}$.4H2O | 0,04 |

Le volume initial (Vi) du fermenteur est ajusté à 13,5 L après ensemencement. Il est porté à 16 - 20 L en final.

Les paramètres de conduite de la fermentation sont les suivants :

Tableau 3.

| Température | 28 °C |
|---|---|
| pH | 5,0 - 5,2 par NH$_3$ 28% p/p |
| pO$_2$ | > 20% (maintenue par agitation) |
| Agitation | 300 RPM mini |
| Débit d'air | 15 L/min |

Lorsque le glucose apporté initialement est consommé, un apport de milieu identique au milieu initial, sans l'antimousse, est réalisé sous forme d'une solution concentrée contenant 500 g/L de glucose et les autres éléments dans les mêmes proportions par rapport au glucose que dans le milieu initial, de façon à obtenir une teneur en glucose de 20 g/L dans le fermenteur.

Deux autres ajouts identiques sont réalisés de la même manière à chaque fois que la concentration résiduelle en glucose devient nulle.

De l'antimousse Clerol FBA 3107 est ajouté à la demande pour éviter un moussage excessif.

Résultats :

Après 46 h de culture, on obtient 38 g/L de biomasse à une teneur en protéines (évalué par le N 6,25) de 36,2 %

**Exemple 2 : Production de *C. sorokiniana* en fermentation de type « fed batch » avec apport en glucose limitant**

Dans cet exemple, un apport de milieu complet (mode « fed-batch ») est démarré après la consommation du glucose apporté initialement. Les autres paramètres de conduite de la fermentation sont inchangés.

Le glucose est apporté en continu à l'aide d'une solution concentrée à 500 g/L. La vitesse d'apport est inférieure à la vitesse de consommation que la souche pourrait réaliser de sorte que la teneur résiduelle en glucose dans le milieu est maintenue nulle, c'est-à-dire que la croissance de la souche est limitée par la disponibilité en glucose (condition glucose-limitant).

Cette vitesse est augmentée au cours du temps de façon exponentielle. La formule servant à calculer le débit d'ajout est caractérisée par un facteur $\mu$ qui correspond au taux de croissance que la souche peut adopter si elle consomme tout le glucose fourni :

$$S = So \times \exp (\mu . t)$$

*S = débit d'apport en glucose (en g/h)*
*So = débit d'apport initial en glucose, déterminé en fonction de la biomasse présente en fin de batch. Il est de 12*

*g/h dans nos conditions.*

*μ = facteur d'accélération du débit. Il doit être inférieur à 0,11 h$^{-1}$ qui est le taux de croissance de la souche en absence de limitation nutritionnelle.*

*t = durée du fed-batch (en h)*

Les sels sont apportés si possible en continu, séparément ou en mélange avec le glucose. Mais ils peuvent être aussi apportés séquentiellement en plusieurs fois.

Le tableau 4 ci-dessous donne les besoins en sels pour 100 g de glucose.

Tableau 4.

| | | |
|---|---|---|
| Macro éléments (g) | Glucose | 100 |
| | $K_2HPO_4.3H2O$ | 6,75 |
| | $MgSO_4.7H2O$ | 1,7 |
| | Ac Citrique | 5,0 |
| | Na2SO4 | 1,0 |
| Micro éléments (mg) | $Na_2EDTA$ | 50 |
| | $CaCl_2.2H2O$ | 400 |
| | $FeSO_4.7H2O$ | 200 |
| | $MnSO_4.4H2O$ | 2,1 |
| | $CoSO_4.7H2O$ | 1,2 |
| | $CuSO_4.5H2O$ | 1,2 |
| | $ZnSO_4.7H2O$ | 2,5 |
| | $H_3BO_3$ | 0,6 |
| | $(NH_4)_6Mo_7O_{27}.4H2O$ | 0,2 |

Les concentrations des autres éléments que le glucose ont été déterminées de façon à être en excès par rapport aux besoins nutritionnels de la souche.

De l'antimousse Clerol FBA 3107 est ajouté à la demande pour éviter un moussage excessif.

Résultats : effet de la vitesse d'apport en glucose durant le fed-batch

Des essais ont été effectués à différentes vitesses d'apport du glucose en « fed-batch ». Ils sont caractérisés par le μ appliqué. La teneur en protéine de la biomasse obtenue est évaluée par la mesure de l'azote total exprimée en N 6.25.

Tableau 5.

| Essai | μ fed (h-1) | Durée (h) | Biomasse (g/L) | Productivité (g/L/h) | % N 6.25 |
|---|---|---|---|---|---|
| 1 | 0,06 | 78 | 43,6 | 0,56 | 49,2 |
| 2 | 0,07 | 54 | 35,1 | 0,65 | 43,1 |
| 3 | 0,09 | 48 | 64,9 | 1,35 | 39,3 |

Ces résultats montrent que le fait de travailler à glucose limitant permet d'augmenter la teneur en protéines.

Il est en effet observé que, même avec un μ élevé de 0,09, on obtient une teneur en protéines plus élevée que celle obtenue sans limitation comme dans l'exemple 1 (39,3 % au lieu de 36,2 %).

Accentuer la limitation du métabolisme par le glucose entraine une amélioration supplémentaire de la teneur en protéines.

Dans les conditions de ces essais, il est nécessaire d'imposer à la souche un μ inférieur à 0,06 h-1 pour obtenir une teneur en protéines supérieure à 50%.

Il est à noter que cette condition va de pair avec une réduction de la productivité : 0,56 g/Uh au lieu de 1,35 g/Uh avec l'essai 3.

**Exemple 3 : Production de C. *sorokiniana* en fermentation continue de type chemostat avec apport de glucose limitant**

Dans cet exemple, le fermenteur utilisé est un 2L de type Sartorius Biostat B.

Il est réalisé comme l'exemple 2, mais avec des volumes 10 fois plus faibles : l'inoculum est de 60 ml et le volume initial de 1,35L.

L'apport en continu du milieu est démarré selon le même principe que dans l'exemple 2, les sels étant dans ce cas mélangés avec le glucose dans la nourrice d'alimentation. La vitesse d'apport est accélérée selon la même formule exponentielle que dans l'exemple 2 en appliquant un $\mu$ de 0,06 h-1.

Chemostat

Lorsqu'un volume de 1,6 L est atteint, soit une concentration en biomasse d'environ 50 g/L, le fonctionnement en continu de type chemostat est mis en place :

  1. Le fermenteur est alimenté en continu à un débit de 96 ml/h par une solution de milieu nutritif à 100 g/L de glucose de composition suivante :

Tableau 6.

| Macro éléments (g/L) | | |
|---|---|---|
| | Glucose | 100 |
| | $K_2HPO_4.3H2O$ | 6,75 |
| | $MgSO_4.7H2O$ | 1,7 |
| | Ac Citrique | 5,0 |
| | Na2SO4 | 1,0 |
| **Micro éléments (mg/L)** | Na$_2$EDTA | 50 |
| | $CaCl_2.2H2O$ | 400 |
| | $FeSO_4.7H2O$ | 200 |
| | $MnSO_4.4H2O$ | 2,1 |
| | $CoSO_4.7H2O$ | 1,2 |
| | $CuSO_4.5H2O$ | 1,2 |
| | $ZnSO_4.7H2O$ | 2,5 |
| | $H_3BO_3$ | 0,6 |
| | $(NH_4)_6Mo_7O_{27}.4H2O$ | 0,2 |

Les concentrations des autres éléments que le glucose ont été déterminé de façon à être en excès par rapport aux besoins nutritionnels de la souche.

  2. Du milieu est soutiré en continu du fermenteur grâce à un tube plongeant relié à une pompe de façon à maintenir le volume de la culture à 1,6L.

Ainsi, le milieu est renouvelé d'une fraction 0,06 (6 %) par heure. Ce taux de renouvellement est appelé taux de dilution (D).

Conformément au principe de la culture en chemostat, le taux de croissance de la souche ($\mu$) s'établit à la même valeur car la croissance de la souche est limitée par l'apport en glucose :

$$D = \mu = 0,06 \text{ h}^{-1}$$

Résultats

Après 97 h de fonctionnement en chemostat, la concentration en biomasse se stabilise à 48 g/L +/- 2 g/L et la teneur en protéine à 53 +/- 2 %.

Ce mode de fonctionnement permet d'obtenir une biomasse à forte teneur en protéine grâce à la limitation en glucose et au faible taux de croissance imposé tout en assurant une très bonne productivité, de l'ordre de 2,9 g/L/h, grâce à la concentration élevée en biomasse.

**Revendications**

1. Procédé d'enrichissement en protéines d'une microalgue cultivée en hétérotrophie, microalgue du genre *Chlorella,* **caractérisé en ce que** la culture hétérotrophique comporte une étape visant à limiter la croissance de ladite microalgue par une carence du milieu de fermentation en glucose avec un apport en glucose en continu en quantité insuffisance de manière à obtenir un taux de croissance de la microalgue inférieur à celui de ladite microalgue en absence de limitation nutritionnelle, et par une augmentation de la température de fermentation par rapport à la température optimale de fermentation, permettant ainsi d'atteindre une teneur en protéines de la biomasse de plus de 50 % en poids.

2. Procédé selon la revendication 1, **caractérisé en ce que** la microalgue du genre Chlorella est choisie dans le groupe constitué de *Chlorella sorokiniana* et *Chlorella protothecoides.*

3. Procédé selon la revendication 2, **caractérisé en ce que** la culture hétérotrophique des microalgues de l'espèce *Chlorella sorokiniana* comprend :

   a. une première étape de croissance des microalgues, où le débit d'apport en glucose est ajusté à leur capacité de consommation, de façon à obtenir rapidement une biomasse importante,
   b. une deuxième étape de production de protéines par les microalgues, où le débit d'apport en glucose est fixé à une valeur nettement en dessous de leur capacité de consommation du glucose, de façon à empêcher l'accumulation supplémentaire de substances de réserve, voire favoriser leur consommation.

4. Procédé selon l'une quelconque des revendications 1 à 3, **caractérisé en ce que** la température de fermentation est augmentée de 1, 2, 3, 4 ou 5°C par rapport à la température optimale de fermentation.

5. Procédé selon la revendication 4, **caractérisé en ce que** la température de fermentation est augmentée d'environ 3°C par rapport à la température optimale de fermentation.

6. Procédé selon l'une quelconque des revendications 1 à 5, **caractérisé en ce que** la température de fermentation est d'environ 31°C.

7. Procédé selon l'une quelconque des revendications 4 à 6, **caractérisé en ce que** l'augmentation de la température de fermentation conduit à augmenter, de préférence doubler, la capacité de refroidissement de l'échangeur thermique du fermenteur.

**Patentansprüche**

1. Verfahren zur Anreicherung von Proteinen einer heterotroph kultivierten Mikroalge der Gattung *Chlorella*, **dadurch gekennzeichnet, dass** die heterotrophe Kultivierung einen Schritt der Limitierung des Wachstums besagter Mikroalge durch einen Glucose-Mangel des Fermentationsmediums bei einer kontinuierlichen Zuführung von Glucose in einer nicht hinreichenden Menge, um eine Wachstumsrate der Mikroalge zu erhalten, die geringer ist als diejenige besagter Mikroalge ohne Nährstofflimitierung, und durch eine Erhöhung der Fermentationstemperatur bezogen auf die optimale Fermentationstemperatur umfasst, wobei das Verfahren folglich erlaubt, einen Proteingehalt der Biomasse von mehr als 50 Gew.-% zu erreichen.

2. Verfahren gemäß Anspruch 1, **dadurch gekennzeichnet, dass** die Mikroalge der Gattung *Chlorella* aus der Gruppe ausgewählt ist, bestehend aus *Chlorella sorokiniana* und *Chlorella protothecoides*.

**3.** Verfahren gemäß Anspruch 2, **dadurch gekennzeichnet, dass** die heterotrophe Kultivierung der Mikroalgen der Spezies *Chlorella sorokiniana* umfasst:

a) einen ersten Schritt des Wachstums der Mikroalgen, in dem die Zuführungsrate von Glucose an ihre Verbrauchskapazität angepasst ist, um eine große Biomasse schnell zu erhalten,
b) einen zweiten Schritt der Produktion von Proteinen von den Mikroalgen, in dem die Zuführungsrate von Glucose auf einen Wert festgesetzt ist, der deutlich unter ihrer Verbrauchskapazität von Glucose liegt, um die zusätzliche Akkumulation von Reservestoffen zu verhindern oder sogar ihren Verbrauch zu fördern.

**4.** Verfahren gemäß einem der Ansprüche 1 bis 3, **dadurch gekennzeichnet, dass** die Fermentationstemperatur um 1, 2, 3, 4 oder 5°C bezogen auf die optimale Fermentationstemperatur erhöht ist.

**5.** Verfahren gemäß Anspruch 4, **dadurch gekennzeichnet, dass** die Fermentationstemperatur ungefähr um 3°C bezogen auf die optimale Fermentationstemperatur erhöht ist.

**6.** Verfahren gemäß einem der Ansprüche 1 bis 5, **dadurch gekennzeichnet, dass** die Fermentationstemperatur ungefähr 31°C beträgt.

**7.** Verfahren gemäß einem der Ansprüche 4 bis 6, **dadurch gekennzeichnet, dass** die Erhöhung der Fermentationstemperatur zu einer Erhöhung, vorzugsweise zu einer Verdoppelung, der Kühlkapazität des Wärmeaustauschers des Fermenters führt.

## Claims

**1.** A process for the protein enrichment of a microalga cultured heterotrophically, the microalga being of the genus *Chlorella*, **characterized in that** the heterotrophic culturing comprises a step directed toward limiting the growth of said microalga via deficiency of the fermentation medium in glucose with a continuous supply in glucose in an insufficient amount so as to obtain a microalgal growth rate below the one of said microalga in absence of nutritional limitation, and by an increase of fermentation temperature relative to the optimum fermentation temperature, thus making it possible to reach a protein content of the biomass of more than 50% by weight.

**2.** The process as claimed in claim 1, **characterized in that** the microalga of the genus Chlorella is chosen from the group constituted by *Chlorella sorokiniana* and *Chlorella protothecoides*.

**3.** The process as claimed in claim 2, **characterized in that** heterotrophic culturing of the microalgae of the species *Chlorella sorokiniana* comprises:

a. a first step of growth of the microalgae, in which the glucose supply rate is adjusted to their consumption capacity, so as rapidly to obtain a large biomass,
b. a second step of protein production by the microalgae, in which the glucose supply rate is set at a value markedly below their glucose consumption capacity, so as to prevent the additional accumulation of storage substances, or even to promote their consumption.

**4.** The process as claimed in any one of claims 1 to 3, **characterized in that** the fermentation temperature is increased by 1, 2, 3, 4 or 5°C relative to the optimum fermentation temperature.

**5.** The process as claimed in claim 4, **characterized in that** the fermentation temperature is increased by about 3°C relative to the optimum fermentation temperature.

**6.** The process as claimed in any one of claims 1 to 5, **characterized in that** the fermentation temperature is about 31°C.

**7.** The process as claimed in any one of claims 4 to 6, **characterized in that** the fermentation temperature increase leads to increasing, preferably doubling, the cooling capacity of the heat exchanger of the fermenter.

## RÉFÉRENCES CITÉES DANS LA DESCRIPTION

**Documents brevets cités dans la description**

- WO 0154510 A

- WO 2014154787 A

**Littérature non-brevet citée dans la description**

- **WANG ; PENG.** *World J Microbiol. Biotechnol.,* 2008, vol. 24 (9), 1915-1922
- **JIANG ; CHEN.** *Process Biochem.,* 2000, vol. 35 (10), 1205-1209

- **XIONG et al.** *Plant Physiology,* 2010, vol. 154, 1001-1011
- **SANSAWA et al.** *Journal of Bioscience and Bioengineering,* 2004, vol. 98, 437-444